# EUROPÄISCHE PATENTSCHRIFT

(11) **EP 1 591 064 B1**
(45) Veröffentlichungstag und Bekanntmachung des Hinweises auf die Patenterteilung: **27.10.2010**
(21) Anmeldenummer: 05008788.1
(22) Anmeldetag: 21.04.2005
(51) Int. Cl.: A61B 5/11

(54) **Messgerät**
measuring device
appareil de mesure

(30) Priorität: 30.04.2004 DE 102004021407
(43) Veröffentlichungstag der Anmeldung: 02.11.2005
(73) Patentinhaber: Rupp + Hubrach Optik GmbH, 96050 Bamberg (DE)
(72) Erfinder: Thiehofe, Ralf, 96163 Gundelsheim (DE); Steinfelder, Hermann, 96050 Bamberg (DE); Störmann, Bernadett, 90542 Eckental (DE); Hausmann, Anne, 96050 Bamberg (DE); Stein, Thorsten, 96135 Stegaurach (DE)
(74) Vertreter: Hofmann, Harald

(56) Entgegenhaltungen:
- EP-A- 1 038 495
- WO-A-2004/008427
- US-A- 4 190 331
- US-A1- 2003 143 391
- PATENT ABSTRACTS OF JAPAN Bd. 2002, Nr. 03, 3. April 2002 (2002-04-03) & JP 2001 314392 A (YAMADA YOSHIAKI; HIRANO HIDETOSHI), 13. November 2001 (2001-11-13)

## Beschreibung

Die vorliegenden Erfindung betrifft allgemein eine Vorrichtung zur Messung des Winkels, zwischen Nahsichtvektor und Fernsichtvektor, insbesondere an einer Brille, und ein Verfahren zur Messung dieses Winkels.

Die Mehrzahl der bisherigen Ansätze zur Anpassung eines Gleitsichtglases an einen konkreten Träger beschränken sich auf die Auswahl eines am Besten passenden Glases aus einer Gruppe/Reihe von Gläsern mit unterschiedlichen Eigenschaften, wie beispielsweise Beabstandung des Fernsichtpunktes vom Nahsichtpunkt und dem dazwischen liegenden Progressionsverlauf.

Aufgrund dieser Vorgehensweise ist das ausgewählte Gleitsichtglas meist nicht optimal auf den Brillenträger angepaßt, insbesondere ist der Abstand des Nahsichtpunktes und des Fernsichtpunktes, der von Bedeutung für den Tragekomfort und die Ergonomie ist, meist nicht optimal an den konkreten Träger angepaßt.

Ein wichtiger Parameter zur Herstellung eines individuellen Gleitsichtglases für einen konkreten Träger ist die individuell unterschiedliche Positionierung des Kopfes im Nahsichtbereich und Fernsichtbereich. Bei Kenntnis dieses Parameters besteht die Möglichkeit Femsichtpunkt und Nahsichtpunkt im Gleitsichtglas für den Träger optimal festzulegen und ein entsprechendes Glas für ihn individuell herzustellen und/oder in der Fassung zu montieren.

Die WO 01 621 39 beschreibt unter anderem eine Meßkammer zur Messung der Kopfposition eines Brillenträgers im Fern- und Nahsichtbereich. Besonders von Nachteil ist bei der durch dieses Dokument vorgeschlagenen Lösung, dass einerseits ein solches System relativ kostenträchtig ist und andererseits die Messung durch die für den Träger ungewohnte Situation verfälscht werden kann. Zudem wird vorgeschlagen, die Kopfposition nur indirekt zu messen, so dass auch hier Fehlerquellen entstehen können.

Die US 4,190,331 beschreibt eine mechanische ophthalmische Messvorrichtung, welche vor den Gläsern eine Brille montiert werden kann, um unter anderem mittels eines Rohres, durch welches der Patient blicken muss, den Blickwinkel zu bestimmen. Diese Vorrichtung ist jedoch mit einer erheblichen Beeinträchtigung des Patienten verbunden.

In der US 2003/0143391 A1 ist eine Vorrichtung zu Messung des Winkels zwischen verschiedenen Sichtvektoren beschrieben. Dabei werden zwei Marker mittels eines Stirnbands am Kopf des Patienten befestigt und die Bewegung der Marker mit einer Videokamera detektiert. Auch diese Anordnung bringt eine erhebliche Beeinträchtigung des Patienten mit sich.

Die WO 2004/008427 A1 beschreibt eine Vorrichtung, welche Personen mit Gleichgewichtsstörungen hilft, indem ein künstlicher Horizont auf einem Brillenglas eingeblendet wird. Die Vorrichtung umfasst auch einen Neigungs- oder Bewegungssensor, welcher an einem Brillenbügel angebracht ist, um Bewegungen des Patienten zu erfassen.

Die US 2002/0021407 A1 beschreibt eine Videospielvorrichtung, welche an einem Brillenbügel angebracht werden kann. Die Videospielvorrichtung umfasst auch einen Bewegungssensor der Neigungs- und Linearbewegungen erfassen kann um die Spielvorrichtung zu Steuern.

Es ist daher Aufgabe der Erfindung, eine Vorrichtung und ein Verfahren anzugeben, das eine einfache, schnelle und kostengünstige Ermittlung des Winkels zwischen der Kopfposition im Fernsichtbereich und der im Nahsichtbereich ermöglicht.

Diese Aufgaben werden durch die Merkmale der unabhängigen Patentansprüche gelöst, wobei zweckmäßige Weiterbildungen durch die Merkmale der abhängigen Ansprüche beschrieben sind.

Insbesondere schlägt die vorliegende Erfindung eine Vorrichtung zur Messung des Winkels zwischen Nahsichtvektor und Femsichtvektor an einer Brille vor, wobei die

Vorrichtung eine in situ Messung, ohne Beeinträchtigung der Physiologie und der Kopfbewegung bereitstellt.

Unter dem Nahsichtvektor bzw. dem Fernsichtvektor ist vorliegend der Normalvektor, der üblicherweise der Erstreckung des Brillenbügels entspricht, entsprechend beim "Sehen in die Ferne" und beim "Sehen in die Nähe" zu verstehen. Durch Messung des von den Vektoren eingeschlossenen Winkels besteht die Möglichkeit ein Gleitsichtglas mit optimalen Eigenschaften für den individuellen Träger herzustellen.

Unter einer in situ Messung ist vorliegend eine Messung direkt am Kopf, insbesondere an der Brille des Trägers zu verstehen.

Optimalerweise wird der Träger einer Brille die Vorrichtung nur unwesentlich wahrnehmen, so dass Meßfehler aufgrund eines besonderen Meßgefühls, einer Einschränkung der Bewegungsfreiheit oder der eigenen Physiologie des Trägers beim Ändern der Kopfposition vom Fern- in den Nahsichtbereich vermieden werden.

Ferner muss der Träger bei einer in situ Messung keine für ihn ungewöhnlich Haltung oder Position einnehmen, die wiederum zu einer fehlerhaften Messung führen könnten.

Vorteilhafterweise ist die Vorrichtung zur Messung an einer beliebigen Brille, insbesondere an einem beliebigen Brillenbügel mittels einer Halterung anordenbar.

Dadurch besteht die Möglichkeit entweder eine einheitliche Meßbrille zu verwenden oder, soweit bereits vorhanden eine Brille des Trägers zu verwenden. Dies hat den Vorteil, dass eventuelle Änderungen des Bewegungsverhaltens mit unterschiedlichen Brillen auf einfache Weise begegnet und vermieden werden können.

Weiterhin ist es von Vorteil, dass die Halterung zumindest auch eine Fixierung der Vorrichtung im wesentlichen am distalen Ende des Brillenbügels bereitstellt. Für eine Fixierung der Vorrichtung kann beispielsweise eine Verrastung, ein Federmechanismus oder eine Fixierschraube verwendet werden, um eine notwendige exakte Positionierung der Vorrichtung während der Messung gewährleisten zu können. Die Anbringung der Vorrichtung möglichst nahe am Ohransatz, also am distalen Ende des Brillenbügels soll zudem eine möglichst geringe Beeinträchtigung der Messung durch das Eigengewicht der Vorrichtung gewährleisten, indem dadurch ein Verkippen der Brille verhindert wird.

Von Vorteil ist weiterhin, dass der Schwerpunkt der Vorrichtung in Anlage an dem Brillenbügel unterhalb des Brillenbügels vorgesehen ist. Dadurch liegt im wesentlichen das gesamte Gewicht der Vorrichtung am Ohr an, ohne dass die Brille verkippt werden würde und die Messung verfälscht werden würde.

Die Vorrichtung umfasst zumindest einen elektronischen Winkelsensor. Es besteht zwar auch die Möglichkeit, beispielsweise einen optischen Sensor zu verwenden, jedoch weisen elektronische Sensoren reltiv geringe Toleranz und geringe Bauraumanforderung auf. Für die Anforderungen an eine exakte Positionierung des Fernsichtpunktes und des Nahsichtpunktes im Gleitsichtglas ist zudem eine Meßauflösung des Sensors von +/- 1 ° (DEG) zu fordern. Die Verwendung eines elektronischen Sensors hat zudem den Vorteil, dass die Meßdaten auf einfache Weise in binärer Form erfaßt werden können, so dass ein weiterer Umwandlungsschritt entfallen kann.

Zudem ist es von Vorteil, dass der Winkelsensor eine Permanentkalibrierung aufweist. Dadurch besteht zum einen die Möglichkeit den Wartungsaufwand und damit die Kosten einer erfindungsgemäßen Vorrichtung möglichst gering zu halten, als auch eventuelle Fehlerquellen im Rahmen der Kalibrierung zu vermeiden.

Weiterhin ist es von Vorteil, dass die Vorrichtung eine Datenübertragungseinrichtung umfasst. Um das Gesamtgewicht der Vorrichtung und damit eine eventuelle Beeinträchtigung der Messung zu verhindern ist es von Vorteil notwendige Anzeigegeräte oder dergleichen nicht in die Vorrichtung zu integrieren, sondern vorteilhafterweise eine kabellose Datenübertragungseinrichtung in die Vorrichtung zu integrieren. Zudem kann mittels des Übertragungskanals auch die Messung ausgelöst werden, ohne dabei die Vorrichtung berühren zu müssen.

In einer bevorzugten Ausführungsform umfasst die Datenübertragungseinrichtung zumindest einen Infrarotsender/-empfänger. Mittels einer Sender/Empfängereinheit können die binären Meßdaten auf einfache Weise auf ein Anzeigegerät übertragen und ausgewertet, als auch die Messung gestartet und beendet werden.

Ferner ist es von Vorteil, dass die Vorrichtung ein Gesamtgewicht von maximal 25 g, insbesondere von maximal 20 g aufweist. Dadurch kann gewährleistet werden, dass der Träger optimalerweise die Vorrichtung an seiner Brille bzw. an einer Meßbrille nicht oder nur unwesentlich wahrnimmt, so dass dadurch die Messung bzw. die Bewegung des Trägers nicht beeinträchtigt wird.

Zur Energieversorgung weist die Vorrichtung ferner einen entsprechenden Kondensator oder dergleichen auf, der mittels eines Berührungskontakts oder Induktion durch eine Ladestation, die optimalerweise in das Anzeigegerät integriert ist, aufgeladen werden kann.

Weiterhin schlägt die vorliegende Erfindung einen Verfahren zur Messung eines Winkels, der von einem Nahsichtvektor und einem Fernsichtvektor eingeschlossen wird vor, wobei folgende Verfahrensschritte vorgesehen sind:
a) Anordnen einer oben beschriebenen Vorrichtung an einer Brille einer Meßperson;
b) Ausrichtung der Kopfposition einer Meßperson im Fernsichtbereich;
c) Schwenken der Kopfposition vom Fernsichtbereich in den Nahsichtbereich;
d) Übertragung der Meßdaten an ein Anzeigegerät;
e) Berechnung und Anzeige des Differenzwinkels durch das Anzeigegerät.

Das Anordnen der Vorrichtung an einem Brillenbügel kann, wie bereits oben beschrieben mittels einer Arretiervorrichtung, wie beispielsweise einer Fixierschraube oder ähnlichem erfolgen.

Im Anschluß daran wird die zu vermessende Person aufgefordert, auf einen bestimmten Punkt in der Ferne zu blicken, und die Messung wird, beispielsweise mittels eines Druckschalters oder eines drahtlosen Signals, ausgelöst. Während der Messung schwenkt die Meßperson ihre Kopfposition vom Fembereich in den Nahbereich, und die Messung wird, wiederum durch einen Taster oder ein drahtloses Signal, beendet.

Im Anschluß an die Messung erfolgt die Auswertung der Meßergebnisse, und der Differenzwinkel wird durch das Display des Anzeigegerätes, welches, wie bereits oben beschrieben, separat vorgesehen ist, um das Gesamtgewicht der Vorrichtung nicht zu erhöhen, dargestellt.

Falls erforderlich kann die Genauigkeit des Ergebnisses weiter erhöht werden, indem die Messung wiederholt wird und eine Mittelwertberechnung erfolgt.

Zudem ist es von Vorteil, wenn der Träger der Korrektionsbrille die Auslösung und Beendigung der Messung selbst vornimmt, da Fern- und Nahsichtbereich individuell unterschiedlich sind; in diesem Fall sollte eine Datenübertragung gewählt werden, die keine dezidierte Ausrichtung erfordert.

Durch ein erfindungsgemäßes Verfahren kann somit auf einfache und schnelle Weise der Differenzwinkel zwischen Nah- und Fernsichtbereich mit hoher Genauigkeit und Reproduzierbarkeit ermittelt werden.

Weitere Merkmale und Vorteile der Erfindung werden deutlicher beim Lesen der folgenden, lediglich beispielhaften und nicht einschränkend angeführten Beschreibung einer bevorzugten Ausführungsform, welche unter Bezugnahme auf die beiliegenden Zeichnungen erfolgt. Darin zeigt:
- Figur 1: eine schematische Ansicht einer Ausgestaltung einer erfindungsgemäße Vorrichtung,
- Figur 2: die Vorrichtung aus Figur 1, die an einem Lade- und Anzeigegerät angeordnet ist,
- Figur 3: eine schematische Ansicht einer erfindungsgemäßen Vorrichtung, an einer Brille eines Trägers angeordnet, beim Sehen in die Ferne,
- Figur 4: eine schematische Ansicht einer erfindungsgemäße Vorrichtung, an einer Brille eines Trägers angeordnet, beim Sehen in die Nähe.

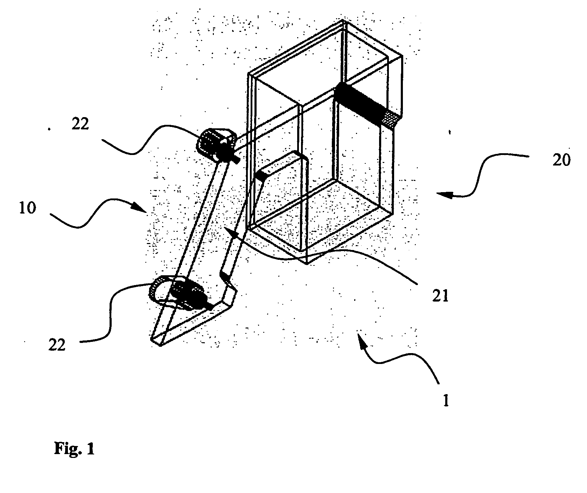

Figur 1 zeigt eine schematische Ansicht einer Ausgestaltung einer erfindungsgemäße Vorrichtung 1, wobei lediglich eine Grundfunktionalität gezeigt ist.

Die Vorrichtung 1 ist im wesentlichen aus einer abstehenden Halterung 10 und einem Gehäuse 20, dass der Aufnahme einer nicht dargestellten Platine dient aufgebaut.

Die nicht dargestellte Platine umfasst dabei einen Winkelsensor, wobei auch ein integrierender Beschleunigungssensor verwendet werden kann. Zudem ist auf der Platine der die Energieversorgung bereitstellende Kondensator bzw. ein entsprechender Schwingkreis, als auch der eine Übertragungsmöglichkeit bereitstellende Infrarotsender/-empfänger, vorgesehen. Der Winkelsensor ist dabei werkseitig dauerhaft kalibriert, so dass weitere Bauteile nicht notwendig sind. Ferner erlaubt der Winkelsensor eine Relativmessung, dh gemessen wird lediglich die Winkeldifferenz, so dass die Messung im wesentlichen unabhängig von der anfänglichen Positionierung der Vorrichtung 1 an dem Brillenbügel ausgeführt werden kann.

Die Halterung 20 weist einen, einer gängigen Bügelform nachgebildeten, mittels Wandungen ausgebildeten Hohlraum 21 zur Aufnahme eines Bügels einer Brille auf.

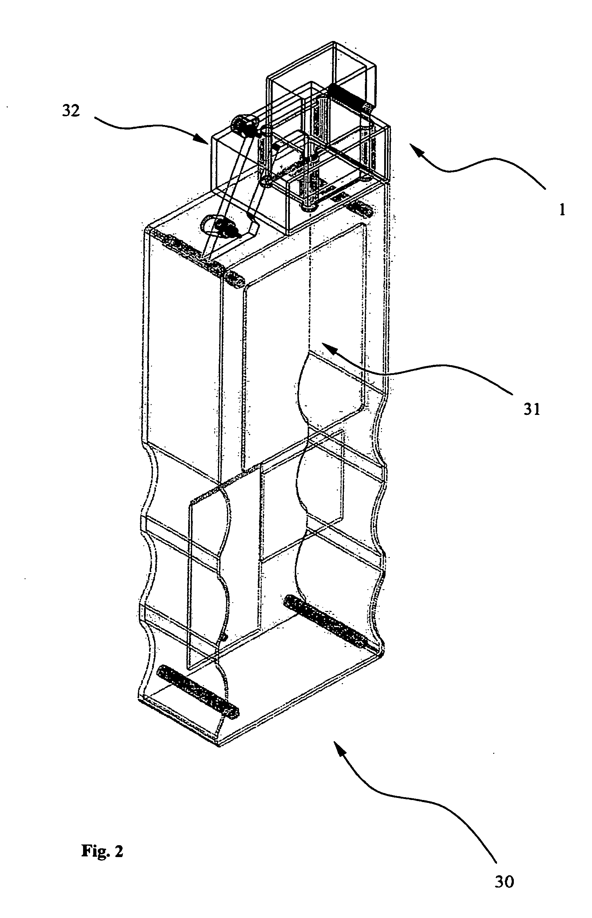

Figur 2 zeigt die Vorrichtung 1 aus Figur 1 an einem Anzeigegerät 30 positioniert, wobei das Anzeigegerät 30 ebenfalls lediglich schematisch dargestellt ist.

Das Anzeigegerät 30 stellt im wesentlichen eine Möglichkeit bereit den Kondensator bzw. den Schwingkreis der Vorrichtung 1 aufzuladen, die vom Infrarotsender/-empfänger emittierten Daten zu Empfangen und visuell darzustellen, als auch die Messung selbst durch den Brillenträger, beispielsweise mittels einer Taste auszulösen.

Das Anzeigegerät 30 umfasst im wesentlichen ein Display 31 und ein nicht näher dargestelltes Batteriefach. An der Oberseite weist das Anzeigegerät 30 eine dem Querschnitt der Vorrichtung 1 entsprechende Aufnahme 32 zur Positionierung der Vorrichtung 1 auf. Während die Vorrichtung 1 in der Aufnahme 32 Positioniert ist, wird der Kondensator durch einen Kontakt bzw. bei Verwendung eines Schwingkreiskondensators induktiv aufgeladen.

Der Kondensator wird dabei in wenigstens einer Minuten vollständig aufgeladen, wobei der Kondensator eine Betriebszeit der Vorrichtung 1 von wenigstens 5 Minuten bereitstellt.

Ferner sind alle Bauteile der Vorrichtung 1 und des Anzeigegerätes 30 EMI/EMP gesichert, so dass keine Störungen von und durch andere Geräte erfolgen, insbesondere nicht mit einem eventuell benachbarten Höhrgerät des Trägers.

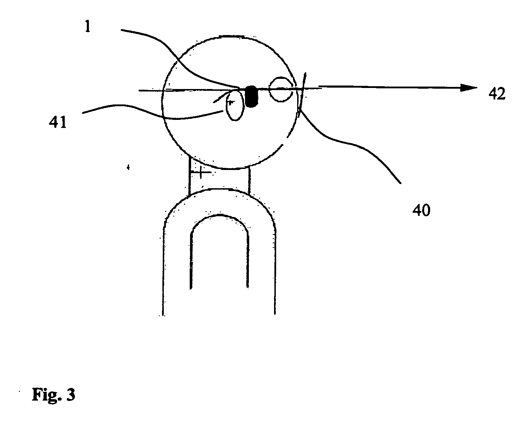

Figur 3 zeigt eine schematische Ansicht einer erfindungsgemäßen Vorrichtung 1, an einer Brille 40 eines Trägers angeordnet, beim Sehen in die Ferne.

Wie in Figur 3 gezeigt wird die Vorrichtung 1 möglichst nahe dem Ohransatz 41 angeordnet, damit das Gewicht der Vorrichtung 1 möglichst geringe Hebelkräfte auf die Brille 40 ausübt und somit die Messung und das Tragegefühl verändert würde.

Die Messung beginnt in der dargestellten Ausführung mit der Kopfpositionierung für den Fernsichtbereich, beispielsweise durch Betätigung einer Taste, die ebenfalls am Anzeigegerät 30 vorgesehen sein kann und mittels drahtloser Übertragung die Meßelektronik der Vorrichtung 1 startet. Der Fernsichtvektor ist beim Bezugszeichen 42 dargestellt.

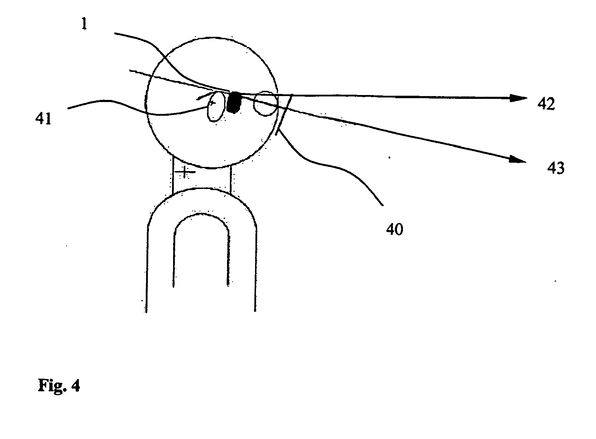

Figur 4 zeigt eine schematische Ansicht einer erfindungsgemäße Vorrichtung 1, an einer Brille 40 eines Trägers angeordnet, beim Sehen in die Nähe. Der Nahsichtvektor ist beim Bezugszeichen 43 dargestellt.

In der in Figur 4 gezeigten Positionierung des Kopfes beendet der Brillenträger die Messung beispielsweise wiederum mittels Betätigung der Taste am Anzeigegerät 30. Dadurch wird ebenfalls die Übertragung der Daten zum Anzeigegerät 30 und die Berechnung des Differenzwinkels zwischen Fern- 42 und Nahsichtvektor 43 ausgelöst und am Display 31 angezeigt.

Zusammenfassend ist festzustellen, daß das erfindungsgemäße Konzept darauf beruht, den Winkel zwischen Nah- und Fernsichtbereich eines Brillenträgers durch ein Meßmittel zu messen, das der Träger während der Messung im wesentlichen nicht wahrnimmt, und er dadurch die Kopfposition in gewohnter Weise verändern kann.

Obwohl die vorliegende Erfindung vorangehend unter Bezugnahme auf die derzeit bevorzugten Ausführungsformen vollständig beschrieben wurden, sollte der Fachmann erkennen, daß verschiedene Veränderungsmöglichkeiten im Rahmen der beiliegenden Ansprüche möglich sind, ohne von dem erfindungsgemäßen Konzept und dem beanspruchten Schutz abzuweichen. Insbesondere kennt der Fachmann auch andere, als die hier vorgeschlagenen Haltevorrichtungen. Ebenfalls kennt der Fachmann Möglichkeiten, die Anzeigefunktion in die Vorrichtung selbst zu integrieren, oder andere Übertragungs- und Auswertungsmöglichkeiten für die Meßdaten zu verwenden. Schließlich sollte der Fachmann erkennen, daß die Vorrichtung und das Verfahren der vorliegenden Erfindung auch anderweitig Anwendung finden kann, z.B. in sogenannten Virtual-Reality- oder Virtualy-Augmented-Reality-Computeranwendungen, zur Bestimmung der Kopfneigung eines Fahradfahrers oder eines anderen Athleten oder eines Sportgerätes und anderen Anwendungen, in denen man eine schnelle, unkomplizierte in situ Neigungsbestimmung wünscht.

## Patentansprüche

1. Anordnung für eine in situ Messung des Winkels zwischen einem Nahsichtvektor (42) und einem Fernsichtvektor (43) ohne Beeinträchtigung der Physiologie und der Kopfbewegung,
mit einer Vorrichtung (1), die zumindest einen elektronischen Winkelsensor umfasst und zur Messung an einem beliebigen Brillenbügel mittels einer Halterung (10) anordenbar ist, wobei der Schwerpunkt der Vorrichtung (1) in Anlage an dem Brillenbügel unterhalb des Brillenbügels vorgesehen ist,
und mit einem Anzeigegerät (30) zur Auswertung der Messdaten und zur Anzeige des Winkels.

2. Anordnung nach Anspruch 1, **dadurch gekennzeichnet, dass** die Halterung (10) zumindest auch eine Fixierung der Vorrichtung (1) im wesentlichen am distalen Ende des Brillenbügels bereitstellt.

3. Anordnung nach Anspruch 1, **dadurch gekennzeichnet, dass** der Winkelsensor eine Permanentkalibrierung aufweist.

4. Anordnung nach einem der Ansprüche 1 bis 3, **dadurch gekennzeichnet, dass** die Vorrichtung (1) und das Anzeigegerät jeweils eine Datenübertragungseinrichtung umfassen.

5. Anordnung nach Anspruch 4, **dadurch gekennzeichnet, dass** die Datenübertragungseinrichtung zumindest einen Infrarotsender/-empfänger umfasst.

6. Anordnung nach einem der Ansprüche 1 bis 5, **dadurch gekennzeichnet, dass** die Vorrichtung (1) ein Gesamtgewicht von maximal 25 g, insbesondere von maximal 20 g aufweist.

7. Verfahren zur Messung eines Winkels, der von einem Nahsichtvektor (42) und einem Fernsichtvektor (43) eingeschlossen wird, **dadurch gekennzeichnet, dass** folgende Verfahrensschritte vorgesehen sind:
a) Anordnen einer Vorrichtung (1) nach einem der Ansprüche 1 bis 6 an einer Brille (40) einer Meßperson;
b) Ausrichtung der Kopfposition der Meßperson im Fernsichtbereich;
c) Schwenken der Kopfposition vom Fernsichtbereich in den Nahsichtbereich;
d) Übertragung der Meßdaten an das Anzeigegerät (30);
e) Berechnung und Anzeige des Differenzwinkels durch das Anzeigegerät (30).

## Claims

1. Arrangement for in-situ measurement of the angle between a near vision vector (42) and a far vision vector (43) without impairing the physiology and the head movement, having a device (1) that comprises at least one electronic angle detector and being positionable at an arbitrarily ear piece of glasses by means of a fixture (10) for measurement purposes, wherein the centre of gravity of the device (1), being in abutment with the ear piece of the glasses being provided below said ear piece of glasses,
and having a display (30) for exploiting the measurement data and for displaying said angle.

2. Arrangement according to claim 1, **characterised in that** the fixture (10) at least also provides for a fixation of the device (1) substantially at the distal end of the ear piece of the glasses.

3. Arrangement according to claim 1, **characterised in that** the angle detector is comprised of a permanent calibration.

4. Arrangement according to any one of claims 1 to 3, **characterised in that** the device (1) and the display each are comprised of data transfer means.

5. Arrangement according to claim 4, **characterised in that** the data transfer means are comprised of at least one infrared emitter/-receiver.

6. Arrangement according to any one of claims 1 to 5, **characterised in that** the device (1) is having a total weight of a maximum of 25g, in particular of a maximum of 20g.

7. Process for measuring an angle, being included by a near vision vector (42) and a far vision vector (43), **characterised by** the following process steps:
a) positioning a device (1) according to any one of claims 1 to 6 on the glasses (40) of a candidate;
b) adjusting the head position of the candidate in the far vision area;
c) pivoting the head position from the far vision area to the near vision area;
d) transmitting measurement data to the display (30);
e) calculating and displaying the differential angle by means of the display (30).

## Revendications

1. Agencement pour une mesure in situ de l'angle entre un vecteur de vision de près (42) et un vecteur de vision de loin (43), sans contrainte physiologique et sans gêne pour le mouvement de tête, avec un dispositif (1), qui comprend au moins un capteur d'angle électronique, et qui peut être disposé pour une mesure au niveau d'une branche de lunette quelconque au moyen d'une attache (10), le centre de gravité du dispositif étant prévu en contact contre la branche de lunette, sous la branche de lunette,
et avec un dispositif de visualisation (30), pour l'évaluation des données de mesure et pour la visualisation de l'angle.

2. Agencement selon la revendication 1, **caractérisé en ce que** l'attache (10) comprend au moins aussi une fixation du dispositif (1) essentiellement au niveau d'une extrémité distale de la branche de lunette.

3. Dispositif selon la revendication 1, **caractérisé en ce que** le capteur d'angle comprend une calibration permanente.

4. Agencement selon l'une quelconque des revendications 1 à 3, **caractérisé en ce que** le dispositif (1) et le dispositif de visualisation comprennent chacun un dispositif de transmission de données.

5. Agencement selon la revendication 4, **caractérisé en ce que** le dispositif de transmission de données comprend au moins un émetteur/récepteur infrarouge.

6. Agencement selon l'une quelconque des revendications 1 à 5, **caractérisé en ce que** le dispositif (1) a un poids total de maximum 25g, en particulier de maximum 20g.

7. Procédé de mesure d'un angle, qui est délimité entre un vecteur de vision de près (42) et un vecteur de vision de loin (43), **caractérisé en ce que** les étapes de procédé suivantes sont prévues :
a) disposer un dispositif (1) selon l'une quelconque des revendications 1 à 6 sur des lunettes (40) d'un candidat ;
b) alignement de la position de tête du candidat dans un domaine de vision de loin ;
c) pivotement de la position de tête depuis le domaine de vision de loin dans le domaine de vision de près ;
d) transmission des données de mesure au dispositif de visualisation (30) ;
e) évaluation et visualisation de l'angle de différence par le dispositif de visualisation (30).
